# EUROPÄISCHE PATENTSCHRIFT

(11) **EP 0 754 679 B1**
(45) Veröffentlichungstag und Bekanntmachung des Hinweises auf die Patenterteilung: **28.10.1998**
(21) Anmeldenummer: 96111542.5
(22) Anmeldetag: 17.07.1996
(51) Int. Cl.: C07C 277/02, C07C 279/14

(54) **Verfahren zur Herstellung von Kreatin oder Kreatin-Monohydrat**
Process for the preparation of creatin or creatine-monohydrate
Procédé de préparation de créatine ou monohydrate de créatine

(30) Priorität: 18.07.1995 DE 19526236
(43) Veröffentlichungstag der Anmeldung: 22.01.1997
(73) Patentinhaber: SKW Trostberg Aktiengesellschaft, 83308 Trostberg (DE)
(72) Erfinder: Weiss, Stefan, Dr., 83308 Trostberg (DE); Krommer, Helmut, 83308 Trostberg (DE)
(74) Vertreter: Huber, Bernhard, Dipl.-Chem.

(56) Entgegenhaltungen:
- DE-C- 494 918
- GB-A- 937 931
- CHEMICAL ABSTRACTS, vol. 99, no. 19, 1983 Columbus, Ohio, US; abstract no. 158796t, XP002016822 & J. LABELLED COMPD. RADIOPHARM., Bd. 20, Nr. 3, 1983, Seiten 377-383, M.M. DALY ET AL:

## Beschreibung

Die vorliegende Erfindung betrifft ein Verfahren zur Herstellung von Kreatin bzw. Kreatin-monohydrat durch Umsetzung von Cyanamid mit Natrium- oder Kaliumsarkosinat.

Kreatin kommt im Muskelgewebe vor und stellt als Kreatinphosphat eine Energiereserve des Muskels dar. Aus diesem Grund wird Kreatin als Nahrungsergänzungsmittel, insbesondere im Sportbereich, verwendet, wobei Kreatin üblicherweise als Kreatin-monohydrat appliziert wird. Kreatin kann aus biologischem Material, z.B. Fleischabfällen, gewonnen werden, was technisch jedoch sehr aufwendig und aus hygienischen Gründen bedenklich ist.

Die Herstellung von Kreatin erfolgt entsprechend dem Stand der Technik durch Reaktion von Cyanamid mit Sarkosin [vgl. Ullmann's Encyclopedia of Industrial Chemistry, 5. Auflage, Band A 12, 552, VCH-Verlagsgesellschaft, Weinheim (1987); Strecker, Jahresbericht über die Fortschritte der Chemie 1868, 686 Anm. 1; Volhard, Zeitschrift für Chemie 1869, 318].

Ein entscheidender Nachteil bei der Herstellung von Kreatin oder Kreatin-monohydrat aus Sarkosin und Cyanamid besteht darin, daß Sarkosin ein sehr teures Ausgangsmaterial darstellt und sich bei Verwendung des teuren Sarkosins keine befriedigenden Ausbeuten erzielen lassen.

Der vorliegenden Erfindung liegt daher die Aufgabe zugrunde, ein Verfahren zur Herstellung für Kreatin bzw. Kreatin-monohydrat zu entwickeln, welches die genannten Nachteile entsprechend dem Stand der Technik nicht aufweist, sondern die Herstellung von Kreatin bzw. Kreatin-monohydrat in wirtschaftlich sinnvoller Ausbeute und guter Reinheit ermöglicht.

Diese Aufgabe wurde erfindungsgemäß dadurch gelöst, daß man Cyanamid mit Natrium- oder Kaliumsarkosinat in Wasser oder einem Gemisch aus Wasser und einem organischen Lösemittel bei einer Temperatur von 20 bis 150°C und einem pH-Wert von 7,0 bis 14,0 umsetzt.

Es hat sich nämlich überraschenderweise gezeigt, daß man auf diese Weise das gewünschte Reaktionsprodukt in guten Ausbeuten und in sehr hoher Reinheit erhalten kann. Dies ist deshalb so überraschend, weil Cyanamid bekanntermaßen im alkalischen Bereich zu Dicyandiamid dimerisiert, wobei das Maximum der Reaktionsgeschwindigkeit bei ca. pH 9,6 liegt. Es ist außerdem bekannt, daß oberhalb von pH 9 neben der Dicyandiamid-Bildung auch gleichzeitig eine Anlagerung von Wasser an Cyanamid unter Bildung von Harnstoff erfolgt. Schließlich ist es auch bekannt, daß Kreatin beim Erhitzen in Wasser unter alkalischen Bedingungen zersetzt wird, wobei u.a. Sarkosin, Harnstoff und Methylhydantoin gebildet werden. Aus diesem Grund konnte nicht damit gerechnet werden, daß mit Hilfe des erfindungsgemäßen Verfahrens gute Ausbeuten und hohe Reinheiten des gewünschten Produktes möglich sind.

Besonders überraschend ist, daß sogar technische Lösungen von Natrium- und Kaliumsarkosinat, die nur eine Reinheit von 85 bis 90 Gew.-% aufweisen und etwa 10 Gew.-% Methyliminodiessigsäure in Form ihrer Salze als Verunreinigung enthalten, zur Herstellung von Kreatin bzw. Kreatin-monohydrat mit den oben angegebenen Vorteilen geeignet sind.

Beim Verfahren der vorliegenden Erfindung werden also die Reaktionskomponenten Cyanamid und Natrium- oder Kaliumsarkosinat bei einer Reaktionstemperatur von 20 bis 150°C, vorzugsweise zwischen 50 und 100°C, gegebenenfalls unter Druck zur Umsetzung gebracht.

Es ist erfindungswesentlich, daß die Umsetzung im alkalischen pH-Bereich von 7,0 bis 14,0, vorzugsweise 9,0 bis 10,0, durchgeführt wird. Der entsprechende pH-Wert wird vorzugsweise mit Hilfe einer anorganischen oder einer organischen Säure eingestellt. Als anorganische Säure (Mineralsäure) kann z.B. Salzsäure verwendet werden, als organische Säure wird vorzugsweise Sarkosin, Essigsäure oder Ameisensäure eingesetzt. Statt dessen kann die pH-Wert-Einstellung jedoch ohne weiteres auch mit aliphatischen Mono-, Di- oder Polycarbonsäuren, insbesondere auch aromatischen Carbonsäuren sowie aliphatischen oder aromatischen Sulfonsäuren erfolgen.

Es ist im Rahmen der vorliegenden Erfindung auch möglich, die pH-Werteinstellung mit Basen, insbesondere NaOH oder KOH vorzunehmen, falls im oberen Bereich des anwendbaren pH-Werts gearbeitet werden soll.

Das Molverhältnis Cyanamid zu Natrium- oder Kaliumsarkosinat kann in weiten Grenzen variiert werden. Vorzugsweise wird dieses Verhältnis auf Werte zwischen 1:4 und 4:1 eingestellt. Besonders bevorzugt werden Cyanamid und das Natrium- oder Kaliumsarkosinat in annähernd äquimolaren Mengen, z.B. 0,9:1 bis 1,25:1 verwendet.

Die Reaktionsdurchführung kann beispielsweise in der Weise erfolgen, daß man eine wäßrige Natrium- oder Kaliumsarkosinat-Lösung vorlegt, mit einer Säure oder Lauge den gewünschten pH-Wert einstellt und Cyanamid in Form einer wäßrigen Lösung (beispielsweise als 50 %-ige wäßrige Lösung) oder in fester kristalliner Form zugibt. Man kann jedoch auch so vorgehen, daß die pH-Wert-Einstellung erst während der Zugabe des Cyanamids erfolgt.

Alternativ können auch Cyanamid und Natrium- oder Kaliumsarkosinat gleichzeitig in das Reaktionsgefäß eingetragen werden, wobei der gewünschte pH-Wert simultan mit Hilfe einer Säure oder Base eingestellt wird.

Als Reaktionsgefäß können unter anderem ein Rührbehälter oder ein Schleifenreaktor benutzt werden. Die Verwendung des Schleifenreaktors empfiehlt sich vor allem dann, wenn die Zugabe von Cyanamid im Natrium- oder Kaliumsarkosinat und die Einstellung des pH-Wertes simultan erfolgen.

Gemäß einer bevorzugten Ausführungsform kann das Natrium- oder Kaliumsarkosinat auch in Form der entsprechenden technischen wäßrigen Lösungen verwendet werden, die vorzugsweise eine Konzentration von 35 bis 40 Gew.-% und einen Reinheitsgehalt von etwa 85 bis 90 Gew.-% aufweisen. Im Gegensatz zu Sarkosin sind die technischen wäßrigen Natriumsarkosinat-Lösungen sehr billig, da es sich hierbei um ein großtechnisches Produkt handelt.

Die erfindungsgemäße Umsetzung erfolgt in sehr einfacher Weise in Wasser oder wäßriger Suspension. Sie kann jedoch auch in wäßrigorganischer Phase, z.B. in Gegenwart eines aliphatischen Alkohols mit 1 bis 5 C-Atomen, vorzugsweise Methanol oder Ethanol, vorgenommen werden. Die Verwendung des organischen Lösemittels erleichtert die Einstellung der gewünschten Reaktionstemperatur, indem man das Reaktionsgemisch auf Rückflußtemperatur erhitzt.

Nach erfolgter Umsetzung, die in der Regel nach 2 bis 5 Stunden beendet ist, kann das feste Reaktionsprodukt mit Hilfe der üblichen Apparaturen wie Zentrifugen, Filterpressen oder Drucknutschen isoliert werden. Zur Reinigung bzw. Herstellung sehr reinen Kreatins oder Kreatin-monohydrats kann das Reaktionsprodukt mit kaltem oder heißem Wasser nachbehandelt werden, z.B. durch Waschen mit Wasser oder Suspendieren in Wasser. Das Reaktionsprodukt kann auch aus Wasser umkristallisiert werden.

Die anschließende Trocknung der feuchten Produkte kann z.B. mit Hilfe bekannter Konvektions- oder Kontakttrockner erfolgen. Als Trockner sind z.B. Kammer-, Tunnel-, Horden-, Band-, Etagen-, Düsen-, Strom-, Wirbelschicht- oder Trommeltrockner geeignet.

Zur Herstellung von wasserfreiem Kreatin wird das Produkt so getrocknet, daß der Wassergehalt unter 1 Gew.-% liegt. Dies kann z.B. durch Trocknen im Vakuum bei 80°C erreicht werden.

Zur Herstellung von Kreatin-monohydrat wird der Trocknungsvorgang bei einem Wassergehalt von etwa 12,1 Gew.-% abgebrochen oder die Trocknungsparameter werden so gewählt, daß der Wassergehalt etwa 12,1 Gew.-% nicht unterschreitet. Kreatin-monohydrat hat einen theoretischen Wassergehalt von 12,08 Gew.-%. Überraschenderweise wurde gefunden, daß die Trocknung von feuchtem Kreatin so gezielt durchgeführt werden kann, daß Kreatin-monohydrat erhalten wird. Hierzu eignet sich z.B. eine Trocknung in einem Hordentrockner bei 40°C und 15 bis 20 mbar oder in einem Trommeltrockner bei 50°C und 180 bis 200 mbar.

Mit Hilfe des erfindungsgemäßen Verfahrens lassen sich Kreatin oder Kreatin-monohydrat in Ausbeuten von 60 bis 90 Gew.-% und in sehr hoher Reinheit, die bis 100 % erreichen kann, herstellen, selbst wenn man von technischem Natrium- oder Kaliumsarkosinat-Lösungen mit einer Reinheit von nur 85 bis 90 Gew.-% ausgeht. Da auch die Raum/Zeit-Ausbeuten des erfindungsgemäßen Verfahrens sehr gut sind, eignet es sich in hervorragender Weise zur Durchführung in technischem Maßstab.

Die nachfolgenden Beispiele sollen die Erfindung näher veranschaulichen.

### Beispiel 1

4625 g (16,7 mol) 40 gew.-%-ige technische wäßrige Natriumsarkosinat-Lösung wurden vorgelegt. Unter äußerer Kühlung mit kaltem Wasser und kräftigem Rühren wurde mit konzentrierter Salzsäure ein pH-Wert von 9,6 (bei 20°C) eingestellt. Es wurde auf 80°C erhitzt. 1548 g (18,4 mol) einer 50 gew.-%-igen wäßrigen Cyanamid-Lösung (SKW-Cyanamid L 500) wurden während 90 Minuten unter starkem Rühren gleichmäßig bei einer Innentemperatur von 80 bis 85°C eingetragen. Nach Beendigung der Cyanamid-Zugabe wurde das Reaktionsgemisch noch 2 Stunden bei einer Innentemperatur von 80°C gerührt. Es wurde abgekühlt und das Reaktionsgemisch 4 Stunden unter Wasserkühlung ausgerührt. Das kristalline, gut filtrierbare Reaktionsprodukt wurde abgesaugt und dreimal mit jeweils 1250 ml Wasser chloridfrei gewaschen. Anschließend wurde noch einmal mit 1250 ml warmen Wasser (40°C) nachgewaschen. Es wurde im Vakuumtrockenschrank bei 40°C und 20 mbar getrocknet. Die Ausbeute betrug 1822 g (73,3 %, bezogen auf Natriumsarkosinat) Kreatin-monohydrat. Gehalt (HPLC): 88,1 % Kreatin (berechnet 87,9 %).

### Beispiel 2

277,7 g (1 mol) einer technischen 40 gew.-%-igen wäßrigen Natriumsarkosinat-Lösung wurden vorgelegt. Es wurde mit konzentrierter Salzsäure ein pH-Wert von 9,6 (bei 20°C) eingestellt und auf 95°C erhitzt. Anschließend wurden 105,1 g (1,25 mol) einer technischen 50 gew.-%-igen wäßrigen Cyanamid-Lösung unter intensivem Rühren in dem Maße zugegeben, daß die Innentemperatur nicht über 95°C anstieg. Nach beendeter Cyanamid-Zugabe wurde noch 1 Stunde bei 95°C erhitzt. Es wurde auf 15°C abgekühlt, der kristalline Niederschlag abgesaugt und zweimal mit je 120 ml Wasser chloridfrei gewaschen. Der Rückstand wurde aus Wasser umkristallisiert und bei 80°C/20 mbar getrocknet. Die Ausbeute betrug 73,4 g (56 %) Kreatin. Die Gehaltsbestimmung mittels HPLC ergab einen Gehalt von 100 % (als Standard wurde reines im Chemikalienhandel erhältliches Kreatin-monohydrat verwendet).

### Beispiel 3

493,2 g (1,78 mol) einer technischen 40 gew.-%-igen wäßrigen Natriumsarkosinat-Lösung und 214 g Wasser wurden in einem mit Rührer, Thermometer und pH-Meter versehenen Vierhalskolben vorgelegt. Mit Essigsäure wurde unter Rühren ein pH-Wert von 9,93 bei 20°C eingestellt. Es wurde auf 70°C erhitzt. Unter intensivem Rühren wurden 138,2 g (1,65 mol) einer 50,2 gew.-%-igen wäßrigen Cyanamid-Lösung (SKW-Cyanamid L 500) bei einer Innentemperatur von 70 bis 72°C mit Hilfe einer Dosierpumpe gleichmäßig während 90 Minuten eingetropft. Nach Beendigung der Cyanamid-Zugabe wurde das Reaktionsgemisch noch 60 Minuten bei einer Innentemperatur von 70°C gerührt. Nach Abkühlen auf 15°C wurde der kristalline, gut filtrierbare Niederschlag abgesaugt und ohne Waschen in 330 g Wasser suspendiert. Die Suspension wurde 1 Stunde bei 20°C gerührt. Der Niederschlag wurde abgesaugt und anschließend ohne Waschen bei 30°C im Vakuumtrockenschrank während 16 Stunden getrocknet. Die Ausbeute betrug 177,7 g (72,2 %) Kreatin-monohydrat.

| | |
|---|---|
| Gehalt (HPLC) | 88,0 % Kreatin (berechnet 87,9 %) |
| Kreatinin (HPLC) | < 200 ppm |
| Dicyandiamid | 220 ppm |
| Wasser (Infrarottrocknungswaage bei 105°C) | 11,9 % (berechnet 12,08 %) |

### Beispiel 4

462,5 g (1,67 mol) einer technischen 40 gew.-%-igen wäßrigen Natriumsarkosinat-Lösung und 200 g Wasser wurden vorgelegt. Mit konzentrierter 98 gew.-%-iger Ameisensäure wurde ein pH von 9,81 (bei 20°C) eingestellt. Es wurde auf 50°C erhitzt. 138,2 g (1,65 mol) SKW-Cyanamid L 500 (technische wäßrige Cyanamid-Lösung mit einem Gehalt von 50,2 Gew.-%) wurden unter kräftigem Rühren bei einer Innentemperatur von 50 bis 52°C während 3 Stunden gleichmäßig zugegeben. Das Reaktionsgemisch wurde noch 1 Stunde bei einer Innentemperatur von 50°C gerührt. Anschließend wurde das Reaktionsprodukt abgesaugt und ohne Waschen in 330 g Wasser suspendiert. Die Suspension wurde 2 Stunden bei 20°C gerührt. Es wurde abgesaugt und der Rückstand zweimal mit 100 ml Wasser gewaschen. Nach Trocknen im Vakuum bei 30°C und 20 mbar wurden 165,2 g (67,1 %) Kreatin-monohydrat erhalten.

| | |
|---|---|
| Gehalt | 88,2 % (HPLC, berechnet 87,9 %) |
| Wasser | 12,1 % (IR-Trocknungswaage, 105°C, berechnet 12,08 %) |

### Beispiel 5

674,8 g (2,43 mol) 40 gew.-%-ige wäßrige Natriumsarkosinat-Lösung wurde vorgelegt. Mit 99 gew.-%-iger Essigsäure wurde bei 70°C ein pH-Wert von 8,5 eingestellt. Nach Zusatz von 500 ml Methanol wurden 491,1 g (2,92 mol) einer 25 gew.-%-igen wäßrigen Cyanamid-Lösung unter kräftigem Rühren bei Rückflußtemperatur innerhalb von 2 Stunden zugegeben. Nach beendeter Zugabe der Cyanamid-Lösung wurde unter Rühren noch 2 Stunden unter Rückfluß erhitzt. Nach Abkühlen auf 15°C wurde der kristalline Niederschlag abgesaugt, zweimal mit je 250 ml Wasser gewaschen und anschließend aus Wasser umkristallisiert. Nach Trocknen im Vakuumtrockenschrank bei 80°C und 15 mbar wurden 176,7 g (48,8 %) wasserfreies Kreatin erhalten.
Reinheit (HPLC) : 99,9 %

### Beispiel 6

Analog dem Beispiel 1 wurde eine 40 %-ige wäßrige Kaliumsarkosinat-Lösung mit Cyanamid umgesetzt. Die Ausbeute betrug 1875 g (75,3 %) reines Kreatin-monohydrat.

## Patentansprüche

1. Verfahren zur Herstellung von Kreatin oder Kreatin-monohydrat,
**dadurch gekennzeichnet,**
daß man Cyanamid mit Natrium- oder Kaliumsarkosinat in Wasser oder einem Gemisch aus Wasser und einem organischen Lösemittel bei einer Temperatur von 20 bis 150°C und einem pH-Wert von 7,0 bis 14,0 umsetzt.

2. Verfahren nach Anspruch 1,
**dadurch gekennzeichnet,**
daß die Umsetzung bei einer Temperatur von 50 bis 100°C erfolgt.

3. Verfahren nach den Ansprüchen 1 oder 2,
**dadurch gekennzeichnet,**
daß man den pH-Wert auf 9,0 bis 10,0 einstellt.

4. Verfahren nach einem der Ansprüche 1 bis 3,
**dadurch gekennzeichnet,**
daß die pH-Wert-Einstellung mit einer anorganischen Säure, vorzugsweise Salzsäure vorgenommen wird.

5. Verfahren nach einem der Ansprüche 1 bis 3,
**dadurch gekennzeichnet,**
daß zur pH-Wert-Einstellung eine organische Säure, vorzugsweise Sarkosin, Essigsäure oder Ameisensäure, verwendet wird.

6. Verfahren nach einem der Ansprüche 1 bis 5,
**dadurch gekennzeichnet,**
daß das Molverhältnis Cyanamid zu Natrium- oder Kaliumsarkosinat auf 1:4 bis 4:1 eingestellt wird.

7. Verfahren nach Anspruch 6,
**dadurch gekennzeichnet,**
daß das Molverhältnis Cyanamid zu Natrium- oder Kaliumsarkosinat 0,9:1 bis 1,25:1 beträgt.

8. Verfahren nach einem der Ansprüche 1 bis 7,
**dadurch gekennzeichnet,**
daß das Natrium- oder Kaliumsarkosinat in Form einer 35 bis 45 gew.-%-igen wäßrigen Lösung eingesetzt wird.

9. Verfahren nach einem der Ansprüche 1 bis 8,
**dadurch gekennzeichnet,**
daß man Cyanamid in Form einer wäßrigen Lösung, insbesondere als 50 gew.-%-ige Lösung, einsetzt.

10. Verfahren nach einem der Ansprüche 1 bis 9,
**dadurch gekennzeichnet,**
daß als organisches Lösemittel ein aliphatischer Alkohol mit 1 bis 5 C-Atomen verwendet wird.

11. Verfahren nach einem der Ansprüche 1 bis 10,
**dadurch gekennzeichnet,**
daß das Reaktionsprodukt zur Reinigung mit kaltem oder heißem Wasser behandelt wird.

12. Verfahren nach einem der Ansprüche 1 bis 11,
**dadurch gekennzeichnet,**
daß das Reaktionsprodukt aus Wasser umkristallisiert wird.

13. Verfahren nach einem der Ansprüche 1 bis 12,
**dadurch gekennzeichnet,**
daß man zur Herstellung von wasserfreiem Kreatin das Reaktionsprodukt bis zu einem Wassergehalt von < 1 Gew.-% trocknet.

14. Verfahren nach einem der Ansprüche 1 bis 12,
**dadurch gekennzeichnet,**
daß man zur Herstellung von Kreatin-monohydrat das Reaktionsprodukt bis zu einem Wassergehalt von etwa 12,1 Gew.-% trocknet.

## Claims

1. Process for the production of creatine or creatine monohydrate,
**wherein**
cyanamide is reacted with sodium or potassium sarcosinate in water or in a mixture of water and an organic solvent at a temperature of 20 to 150°C and a pH value of 7.0 to 14.0.

2. Process as claimed in claim 1,
**wherein**
the reaction takes place at a temperature of 50 to 100°C.

3. Process as claimed in claim 1 or 2,
**wherein**
the pH value is adjusted to 9.0 to 10.0.

4. Process as claimed in one of the claims 1 to 3,
**wherein**
the pH value is adjusted with an inorganic acid and preferably with hydrochloric acid.

5. Process as claimed in one of the claims 1 to 3,
**wherein**
the pH value is adjusted with an organic acid and preferably with sarcosine, acetic acid or formic acid.

6. Process as claimed in one of the claims 1 to 5,
**wherein**
the molar ratio of cyanamide to sodium or potassium sarcosinate is adjusted to 1:4 to 4:1.

7. Process as claimed in claim 6,
**wherein**
the molar ratio of cyanamide to sodium or potassium sarcosinate is 0.9:1 to 1.25:1.

8. Process as claimed in one of the claims 1 to 7,
**wherein**
the sodium or potassium sarcosinate is used in the form of a 35 to 45 % by weight aqueous solution.

9. Process as claimed in one of the claims 1 to 8,
**wherein**
the cyanamide is used in the form of an aqueous solution, in particular as a 50 % by weight aqueous solution.

10. Process as claimed in one of the claims 1 to 9,
**wherein**
an aliphatic alcohol containing 1 to 5 carbon atoms is used as the organic solvent.

11. Process as claimed in one of the claims 1 to 10,
**wherein**
the reaction product is purified by treatment with cold or hot water.

12. Process as claimed in one of the claims 1 to 11,
**wherein**
the reaction product is recrystallized from water.

13. Process as claimed in one of the claims 1 to 12,
**wherein**
for the production of anhydrous creatine, the reaction product is dried to a water content of < 1% by weight.

14. Process as claimed in one of the claims 1 to 12,
**wherein**
for the production of creatine monohydrate, the reaction product is dried to a water content of about 12.1 % by weight.

## Revendications

1. Procédé de préparation de créatine ou de monohydrate de créatine, caractérisé en ce que l'on fait réagir du cyanamide avec du sarcosinate de sodium ou de potassium dans l'eau ou dans un mélange d'eau et d'un solvant organique, à une température de 20 à 150°C et à un pH de 7,0 à 14,0.

2. Procédé selon la revendication 1, caractérisé en ce que la réaction s'effectue à une température de 50 à 100°C.

3. Procédé selon les revendications 1 ou 2, caractérisé en ce que l'on règle le pH à une valeur de 9,0 à 10,0.

4. Procédé selon l'une des revendications 1 à 3, caractérisé en ce que le réglage du pH s'effectue à l'aide d'un acide inorganique, de préférence l'acide chlorhydrique.

5. Procédé selon l'une des revendications 1 à 3, caractérisé en ce que, pour régler le pH, on utilise un acide organique, de préférence la sarcosine, l'acide acétique ou l'acide formique.

6. Procédé selon l'une des revendications 1 à 5, caractérisé en ce que l'on règle le rapport molaire du cyanamide au sarcosinate de sodium ou de potassium à une valeur de 1:4 à 4:1.

7. Procédé selon la revendication 6, caractérisé en ce que le rapport molaire du cyanamide au sarcosinate de sodium ou de potassium est compris entre 0,9:1 et 1,25:1.

8. Procédé selon l'une des revendications 1 à 7, caractérisé en ce que l'on utilise le sarcosinate de sodium ou de potassium sous forme d'une solution aqueuse à 35 à 45 % en masse.

9. Procédé selon l'une des revendications 1 à 8, caractérisé en ce que l'on utilise du cyanamide sous forme d'une solution aqueuse, en particulier d'une solution à 50 % en masse.

10. Procédé selon l'une des revendications 1 à 9, caractérisé en ce que l'on utilise comme solvant organique un alcool aliphatique de 1 à 5 atomes de carbone.

11. Procédé selon l'une des revendications 1 à 10, caractérisé en ce que, pour le purifier, on traite le produit réactionnel avec de l'eau froide ou chaude.

12. Procédé selon l'une des revendications 1 à 11, caractérisé en ce que l'on recristallise le produit réactionnel dans l'eau.

13. Procédé selon l'une des revendications 1 à 12, caractérisé en ce que, pour préparer de la créatine anhydre, on sèche le produit réactionnel jusqu'à une teneur en eau inférieure à 1 % en masse.

14. Procédé selon l'une des revendications 1 à 12, caractérisé en ce que, pour préparer le monohydrate de créatine, on sèche le produit réactionnel jusqu'à une teneur en eau d'environ 12,1 % en masse.
